# EUROPEAN PATENT SPECIFICATION

(11) **EP 3 306 313 B1**
(45) Date of publication and mention of the grant of the patent: **15.07.2020**
(21) Application number: 16803123.5
(22) Date of filing: 24.05.2016
(51) Int. Cl.: G01N 33/15, A01K 67/027, A61K 31/282, A61K 31/337, A61K 31/4745, A61K 45/00, G01N 33/50

(54) **METHOD FOR EVALUATING HEMATOLOGICAL TOXICITY OF DRUG UNDER EVALUATION**
VERFAHREN ZUR BEURTEILUNG DER HÄMATOLOGISCHEN TOXIZITÄT EINES ZU BEURTEILTEN ARZNEIMITTELS
PROCÉDÉ PERMETTANT D'ÉVALUER LA TOXICITÉ HÉMATOLOGIQUE D'UN MÉDICAMENT EN COURS D'ÉVALUATION

(30) Priority: 29.05.2015 JP 2015110299
(43) Date of publication of application: 11.04.2018
(73) Proprietor: Central Institute For Experimental Animals, Kawasaki-shi, Kanagawa 210-0821 (JP)
(72) Inventor: OKAMOTO, Kazuya, Tokyo 115-8588 (JP); NAGAI, Daichi, Tokyo 115-8588 (JP); ITO, Mamoru, Kawasaki-shi Kanagawa 210-0821 (JP); ITO, Ryoji, Kawasaki-shi Kanagawa 210-0821 (JP)
(74) Representative: Graf von Stosch, Andreas
(86) International application number: PCT/JP2016/065235
(87) International publication number: WO 2016/194688

(56) References cited:
- WO-A1-2014/087863
- R. ITO ET AL: "Establishment of a Human Allergy Model Using Human IL-3/GM-CSF-Transgenic NOG Mice", THE JOURNAL OF IMMUNOLOGY, vol. 191, no. 6, 15 September 2013 (2013-09-15), pages 2890-2899, XP055508668, US ISSN: 0022-1767, DOI: 10.4049/jimmunol.1203543
- MASAYUKI TAKAHASHI ET AL: "Assessment of Benzene-Induced Hematotoxicity Using a Human-Like Hematopoietic Lineage in NOD/Shi-scid/IL-2R gamma null Mice", PLOS ONE, PUBLIC LIBRARY OF SCIENCE, US , vol. 7, no. ISS.12 1 December 2012 (2012-12-01), pages E50448-1, XP003031931, ISSN: 1932-6203, DOI: 10.1371/JOURNAL.PONE.0050448 Retrieved from the Internet: URL:http://www.plosone.org/article/fetchOb ject.action [retrieved on 2012-12-03]
- ITO R ET AL: "A novel in vivo model for predicting myelotoxicity of chemotherapeutic agents using IL-3/GM-CSF transgenic humanized mice", TOXICOLOGY LETTERS, vol. 281, 13 September 2017 (2017-09-13), pages 152-157, XP085227808, ISSN: 0378-4274, DOI: 10.1016/J.TOXLET.2017.09.013
- MASAYUKI TAKAHASHI ET AL.: 'Development of novel system for health risk assessment using humanized mice(part 2)Application of humanized mice to the assessment of hematotoxicity' CENTRAL RESEARCH INSTITUTE OF ELECTRIC POWER INDUSTRY KANKYO KAGAKU KENKYUSHO KENKYU HOKOKU vol. 11063, January 2013, XP009507730
- ITO RYOJI ET AL. PROCEEDINGS OF THE JAPANESE SOCIETY FOR IMMUNOLOGY vol. 40, 07 November 2011, page 48, XP009507709
- MAMORU ITO: 'Hito-ka Mouse Soshutsu o Mezashita Men'eki Fuzen Mouse no Kaihatsu Kenkyu to sono Rekishi' JAPANESE ASSOCIATION FOR LABORATORY ANIMAL SCIENCE SOKAI KOEN YOSHISHU vol. 62, 30 April 2015, page 57, XP009507711

## Description

### Technical Field

The present invention relates to a method for evaluating hematotoxicity of a drug to be evaluated.

### Background Art

Hematotoxicity such as reduction in leucocytes developed due to myelopathy is a primary side effect of anticancer agents (see NPL 1) and is a dose limiting factor (DLF) in many anticancer agents. In a phase during which a drug is studied and developed, hematotoxicity is evaluated by a hematology inspection using the peripheral blood of an experimental animal. However, when the experimental animal is used to evaluate an anticancer agent for hematotoxicity in the experiment, a degree of myelopathy of experimental animals may be different from a degree of myelopathy of humans, resulting in poor clinical predictability. Particularly, reactivity and a recovery period from the myelopathy obtained from the experiments using rodents are different from clinical findings obtained from the experiments using humans. A factor causing this difference is as follows. Specifically, humans are different from experimental animals in terms of sensitivity of bone marrow cells to anticancer agents and time for proliferating cells. In addition, in order to examine an influence of the anticancer agent on an ability of differentiation into the hematopoietic cells to be proliferated, the colony forming test or ATP assay may be performed. However, these *in vitro* experiments have difficulty in evaluation and are also difficult to examine a change of property of recovering in a case where the anticancer agent develops its effect in the body through metabolism.

In the existing animal experiments, the clinical predictability with respect to toxicity to the bone marrow, which causes the myelopathy, is low. In addition, it is difficult to estimate a dose for the human employed in the clinical practice based on a dose used in the animal experiment. Therefore, there has not been reported an evaluation method that can determine clinical predictability with respect to toxicity to the bone marrow in a highly reliable manner.

A mouse obtained in the following manner has been reported. Specifically, a NOG mouse; i.e., a heavily immunodeficient mouse (see PTL 1 and NPL 2) is systemically irradiated with X-rays to destroy the bone marrow. Then, CD34⁺ cells derived from the human cord blood are transplanted into the NOG mouse (see NPL 3).

Previous reports say that the mouse, which is created by systemically irradiating the NOG mouse with X-rays to destroy the bone marrow and transplanting CD34⁺ cells derived from the human cord blood into the NOG mouse, and the mouse, which is created by systemically irradiating the NOG mouse with X-rays to destroy the bone marrow and transplanting Lineage-cells derived from the bone marrow of the C57BL/6J mouse into the NOG mouse, are evaluated for hematotoxicity using benzene (see NPL 4 and NPL 5).

However, in the humanized NOG mouse, the human leucocytes-based progenitor cells and the human leucocyte-based mature cells are observed in the cells of hemocytes derived from all of the tissues. Meanwhile, differentiation of the myelocyte-based cells is not sufficient. In particular, differentiation into the granulocytes is hardly observed. Therefore, even when the NOG mouse is used, it is difficult to evaluate hematotoxicity of the drug to be evaluated with high clinical predictability of toxicity to the bone marrow. Therefore, there has been no model animal suitable for evaluating the drug to be evaluated for hematotoxicity.

### Citation List

### Patent Literature

PTL 1 Japanese Patent No. 3753321

### Non-Patent Literature

NPL 1 Folia Pharmacologica Japonica 132, p. 343 to 346 (2008)
NPL 2 Blood 100, p. 3175 to 3182 (2002)
NPL 3 Central Research Institute of Electric Power Industry, Research Report: V09011, April 2010
NPL 4 Central Research Institute of Electric Power Industry, Research Report: V11063, January 2013
NPL 5 PLoS One. 2012; 7 (12): e50448

### Summary of Invention

### Technical Problem

An object of the present invention is to solve the existing problems in the art and to achieve the following object. That is, an object of the present invention is to provide: a method for evaluating hematotoxicity of a drug to be evaluated where the method is excellent in clinical predictability of toxicity to the bone marrow.

### Solution to Problem

As a result of the studies diligently performed by the present inventors to solve the aforementioned object, the present inventors have found that a humanized, transgenic NOG mouse can be used to evaluate hematotoxicity to human caused by a drug to be evaluated and have completed the present invention.

The present invention is based on the findings obtained by the present inventors and means for solving the above problem are as follows. That is,
<1> A method for evaluating hematotoxicity of a drug to be evaluated, the method including:
   administering the drug to be evaluated to a humanized, transgenic NOG mouse where the humanized, transgenic NOG mouse is obtained by introducing a human GM-CSF/IL-3 gene combination into a NOG mouse and the number of human-derived leucocytes in leucocytes in blood thereof is 20% or more; and
   evaluating that the drug to be evaluated has the hematotoxicity in a case where when a sample of the humanized, transgenic NOG mouse after the administering the drug to be evaluated is measured for the number of cells of human-derived granulocytes, the number of said granulocytes is reduced compared to a control to which a vehicle is administered instead of the drug to be evaluated.
<2> The method for evaluating the hematotoxicity of the drug to be evaluated according to <1>, wherein the drug to be evaluated is an anticancer agent.
<3> The method for evaluating the hematotoxicity of the drug to be evaluated according to <2>, wherein the anticancer agent is a cytocide-type anticancer agent.
<4> The method for evaluating the hematotoxicity of the drug to be evaluated according to <3>, wherein the cytocide-type anticancer agent is at least one of camptothecin analogues, composites including a camptothecin analogue therein, paclitaxel analogues, composites including paclitaxel therein, and platinum complex derivatives.
<5> The method for evaluating the hematotoxicity of the drug to be evaluated according to <3>, wherein the cytocide-type anticancer agent is at least one of irinotecan, nogitecan, 7-ethyl-10-hydroxycamptothecin, paclitaxel, and oxaliplatin.
<6> The method for evaluating the hematotoxicity of the drug to be evaluated according to any one of <1> to <5>, the method further including:
   measuring a period from when the number of the human-derived granulocytes is reduced compared to the control until when it recovers to the number before the administering the drug, and evaluating property of recovering based on the period.
<7> The method for evaluating the hematotoxicity of the drug to be evaluated according to any one of <1> to <6>, the method further including:
   measuring the numbers of mouse-derived granulocytes and evaluating that the drug to be evaluated has the hematotoxicity in a case where when the number of the cells of the mouse-derived granulocytes is reduced compared to the control to which the vehicle is administered instead of the drug to be evaluated; and
   evaluating a difference of hematotoxicity of the drug to be evaluated between species based on an evaluation result of the cells of the human-derived granulocytes and an evaluation result of the cells of the mouse-derived granulocytes.

### Advantageous Effects of Invention

According to the present invention, it is possible to provide a method for evaluating hematotoxicity of a drug to be evaluated where the method is excellent in clinical predictability of toxicity to the bone marrow, which can solve the existing problems in the art and can achieve the aforementioned object.

### Brief Description of Drawings

FIG. 1 is a graph presenting a change of the number of the human-derived leucocytes (hCD45⁺) in the evaluation of hematotoxicity of nogitecan.
FIG. 2 is a graph presenting a change of the number of the mouse-derived leucocytes (mCD45⁺) in the evaluation of hematotoxicity of nogitecan.
FIG. 3 is a graph presenting a change of the number of the human-derived granulocytes (CD66b⁺) in the evaluation of hematotoxicity of nogitecan.
FIG. 4 is a graph presenting a change of the number of the mouse-derived granulocytes (Gr-1⁺) in the evaluation of hematotoxicity of nogitecan.
FIG. 5 is a graph presenting a change of the human-derived leucocytes (hCD45+) in the evaluation of hematotoxicity of paclitaxel.
FIG. 6 is a graph presenting a change of the number of the mouse-derived leucocytes (mCD45+) in the evaluation of hematotoxicity of paclitaxel.
FIG. 7 is a graph presenting a change of the human-derived granulocytes (CD66b+) in the evaluation of hematotoxicity of paclitaxel.
FIG. 8 is a graph presenting a change of the number of the mouse-derived granulocytes (Gr-1⁺) in the evaluation of hematotoxicity of paclitaxel.
FIG. 9 is a graph presenting a change of a rate between the number of the human-derived leucocytes (hCD45⁺) and the number of the mouse-derived leucocytes (mCD45⁺) of the control group in the evaluation of hematotoxicity of paclitaxel.
FIG. 10 is a graph presenting a change of a rate between the number of the human-derived leucocytes (hCD45⁺) and the number of the mouse-derived leucocytes (mCD45⁺) of the paclitaxel-administered group in the evaluation of hematotoxicity of paclitaxel.
FIG. 11 is a graph presenting a change of human-derived leucocytes (hCD45⁺) in the evaluation of hematotoxicity of oxaliplatin.
FIG. 12 is a graph presenting a change of the number of the mouse-derived leucocytes (mCD45⁺) in the evaluation of hematotoxicity of oxaliplatin.
FIG. 13 is a graph presenting a change of the number of the human-derived granulocytes (CD66b⁺) in the evaluation of hematotoxicity of oxaliplatin.
FIG. 14 is a graph presenting a change of the number of the mouse-derived granulocytes (Gr-1⁺) in the evaluation of hematotoxicity of oxaliplatin.

### Description of Embodiments

### (Method for evaluating hematotoxicity of drug to be evaluated)

A method of the present invention for evaluating hematotoxicity of a drug to be evaluated according to claim 1 includes a step of administering a drug and a step of evaluation, and further includes other steps such as a step of evaluating property of recovering and a step of evaluating a difference between species, if necessary.

The method disclosed herein for evaluating hematotoxicity of the drug to be evaluated may be a method for evaluating *in vivo* hematotoxicity or may be a method for evaluating *in vitro* hematotoxicity.

### <Step of administering drug>

The step of administering the drug is a step of administering a drug to be evaluated to a humanized, transgenic NOG mouse.

### -Humanized, transgenic NOG mouse (model for evaluating hematotoxicity of drug to be evaluated)-

The humanized, transgenic NOG mouse is a transgenic NOG mouse where the number of human-derived leucocytes in leucocytes in blood thereof is 20% or more.

Here, "humanized" means that human-derived blood cells are introduced into the blood.

A model for evaluating hematotoxicity of a drug to be evaluated is another aspect of the disclosure, wherein the model includes the humanized, transgenic NOG mouse.

A model animal of the present disclosure for evaluating hematotoxicity of a drug to be evaluated can suitably be used for the method for evaluating hematotoxicity of the drug to be evaluated.

### --Transgenic NOG mouse (Tg-NOG mouse)--

The transgenic NOG mouse (hereinafter may be referred to as "Tg-NOG mouse") is a transgenic NOG mouse (Tg-NOG mouse) obtained by introducing a human GM-CSF/IL-3 gene into a NOG mouse. Specifically, the transgenic NOG mouse is a transgenic-type NOG mouse, which is obtained by backcrossing the NOG mouse with a C57BL/6 transgenic mouse into which a GM-CSF/IL-3 gene is introduced (C57BL/6-IL-3/GM-CSF-Tg mouse). In addition, the transgenic NOG mouse systemically yields human GM-CSF and human IL-3.

The transgenic NOG mouse is established by Central Institute for Experimental Animals and is a hybrid-type immunodeficient mouse described in Journal of Immunology, 2013, 191: 2890-2899. The transgenic NOG mouse is represented by an official name of "NOD. Cg-Pkdc^{scid} Il2rg^{tm1Sug} Tg (SV40-CSF2, IL3) 10-7 Jic/Jic" and an abbreviated name of "NOG-GM-CSF/IL-3-Tg" and is available from Central Institute for Experimental Animals.

### ---NOG mouse---

The NOG mouse is established by Central Institute for Experimental Animals and is a hybrid-type immunodeficient mouse described in Blood, 2002, 100, 3175-3182 and Japanese Patent No. 3753321. The NOG mouse is represented by an official name of "NOD. Cg-Prkdc^{scid} Il2rg^{tm1Sug}/Jic" and an abbreviated name of "NOD/Shi-scid, IL-2Rγ^{null}" and is available from Central Institute for Experimental Animals.

The NOG mouse is a genetically modified mouse, which is obtained by crossbreeding a NOD/scid mouse and an IL-2 receptor γ-chain knockout (IL2RyKO) mouse and lacks T cells, B cells, and NK cells. Here, the NOD/scid mouse is obtained by modifying an immunodeficient SCID mouse and the IL-2 receptor γ-chain is the common domain for several cytokine receptors.

The NOG mouse has more excellent engraftment of xenogeneic cells compared to the existing immunodeficient mice. For example, when the human hematopoietic stem cells are transplanted, it is demonstrated that differentiation into the human leucocytes can be observed and differentiation into the human T-lymphocytes, which cannot be observed in the NOD/scid mouse, can be also observed. Note that, in the NOG mouse, differentiation of the myelocyte-based cells is not sufficient and an efficiency of differentiation into the granulocytes is considerably low.

Meanwhile, in the transgenic NOG mouse, when the human hematopoietic stem cells are transplanted (to be humanized), efficiency of differentiation of human myelocyte cells increases compared to the NOG mouse. In addition, differentiation into the cells of the human hemocytes, which is significantly useful, is demonstrated. Specifically, differentiation into the human granulocytes, which has not been observed before, is recognized. Based on these findings, the present inventors found that the humanized, transgenic NOG mouse is useful for evaluating hematotoxicity by evaluating reduction in the granulocytes.

The humanized, transgenic NOG mouse can be created by irradiating the transgenic NOG mouse with radiation to eliminate mouse-derived bone marrow cells and transplanting human-derived hCD34⁺ cells into the transgenic NOG mouse to be engrafted.

Examples of the radiation include X-rays and γ-rays.

A method of the irradiation is not particularly limited and may be selected from known methods depending on the intended purpose.

The radiation to be irradiated is preferably 2.0 Gy to 3.0 Gy as an absorbed dose of the X-rays. A frequency of the radiation to be irradiated is not particularly limited and may be appropriately selected depending on the intended purpose.

After irradiation of the radiation, it may be confirmed that the bone marrow cells derived from the transgenic NOG mouse are eliminated.

It may be confirmed by, for example, the following method that the bone marrow cells derived from the transgenic NOG mouse are eliminated.

After the irradiation of the radiation (for example, from immediately after the irradiation of radiation to 3 days after the irradiation of radiation), the transgenic NOG mouse is euthanized by removing the blood from the abdominal aorta under anesthesia of isoflurane. Then, the femur is collected. The femur is washed using the Iscove's Modified Dulbecco's Medium (IMDM medium) to prepare a suspension of cells. The suspension of cells is passed through a nylon filter to obtain a filtrate as a solution of the bone marrow cells. The total number of the leucocytes in the solution of the bone marrow cells is measured by, for example, an automated hematology analyzer. Then, the number of cells of the bone marrow cells derived from the mouse (for example, mouse-derived mCD11b⁺) in the solution of the bone marrow cells is calculated based on ratios obtained through fluorescent flow cytometry (FCM) measurement using a surface antigen marker as an indicator.

When the human-derived hCD34⁺ cells, which are hematopoietic stem cells, are transplanted into the transgenic NOG mouse to be engrafted, the cells of the human-derived hemocytes (for example, human-derived leucocytes hCD45⁺ cells and human-derived granulocytes hCD66b⁺ cells), which are differentiated from the hCD34⁺ cells, will appear in the blood.

A method for transplanting the human-derived hCD34⁺ cells is not particularly limited and may be appropriately selected depending on the intended purpose.

The human-derived hCD34⁺ cells to be transplanted are not particularly limited and may be appropriately selected depending on the intended purpose. Preferable examples thereof include hCD34⁺ cells derived from the human cord blood. The hCD34⁺ cells derived from the human cord blood may be a commercially available product and may be a product isolated from the cord blood of a healthy subject.

The number of the human-derived hCD34⁺ cells to be transplanted is not particularly limited and may be appropriately selected depending on the intended purpose, but is preferably from 1×10⁴ (cells/body) to 1×10⁶ (cells/body).

As a method for confirming engraftment of the cells of the human-derived hemocytes, a method described below can be performed, for example.

Specifically, after the transplantation (from week 5 to week 7 after the transplantation) of the human-derived hCD34⁺ cells, the blood of the transgenic NOG mouse is collected from the orbital sinus. The total number of the leucocytes in the blood is measured by, for example, an automated hematology analyzer. Then, the number of the cells of the human-derived hemocytes (for example, human-derived leucocytes hCD45⁺ cells) and the number of the cells of the mouse-derived hemocytes (for example, mouse-derived leucocytes mCD45⁺ cells) in the blood are calculated based on ratios obtained through fluorescent flow cytometry (FCM) measurement using a surface antigen marker as an indicator.

A ratio between the cells of the mouse-derived leucocytes mCD45⁺ and the cells of the human-derived leucocytes hCD45⁺ is calculated. When the number of the human-derived leucocytes in the leucocytes in the blood thereof is 20% or more, it is judged that the cells of the human-derived hemocytes are engrafted and the transgenic NOG mouse is humanized.

Note that, the confirmation that the cells of the human-derived hemocytes are engrafted may be performed at the same time as the measurement of the number of the cells of the human-derived granulocytes before the administration of the drug to be evaluated.

On the other hand, when the number of the human-derived leucocytes in the leucocytes in the blood is less than 20%, the number of the human-derived granulocytes generated is too small (the number of the granulocytes is about 5% in the leucocytes). Therefore, the aforementioned number of less than 20% is insufficient as the property of the humanized, transgenic NOG mouse of the present invention having a high efficiency of humanization, which is not suitable for evaluating hematotoxicity.

### -Drug to be evaluated-

The drug to be evaluated is not particularly limited and may be appropriately selected depending on the intended purpose. Examples thereof include anticancer agents, antibacterial drugs, anticonvulsant drugs, and antithyroid drugs. Among them, anticancer agents are preferable. In addition, the drug to be evaluated may be any test compounds that are candidates of therapeutic drugs, test drugs, and pharmaceutical agents. Moreover, the drug to be evaluated may be a natural compound or a synthesized compound.

### --Anticancer agent--

The anticancer agent means a drug used for the purpose of inhibiting increase in cancer. Examples thereof include molecular targeting drugs and cytocide-type anticancer agents.

Here, the "cancer" means, in a broad sense, a malignant tumor including: carcinoma that is a malignant tumor derived from an epithelial tissue; sarcoma that is a malignant tumor derived from a non-epithelial tissue; and hematologic malignancy (e.g., leukemia).

### ---Molecular targeting drug---

The molecular targeting drug is a drug that acts on molecules (for example, proteins and genes) specifically expressed in cancer cells as a target. Examples thereof include: an anticancer agent inhibiting signal transduction of the growth factor of cancer cells; an anticancer agent inhibiting a function of, for example, proteins where the proteins are expressed in cancer cells and are related to proliferation of the cancer cells; and an anticancer agent inhibiting proliferation of cancer cells via an immune system, in terms of mechanism of action.

Examples of the molecular targeting drug include a small molecule inhibitor and an antibody drug.

Examples of the small molecule inhibitor include a tyrosine kinase inhibitor, a Raf kinase inhibitor, a proteasome inhibitor, an mTOR inhibitor, a calcineurin inhibitor, and a Janus kinase inhibitor.

Examples of the antibody drug include anti-CD20 antibody, anti-EGFR antibody, anti-TNF-α antibody, anti-CD30 antibody, anti-human IL-6 receptor antibody, anti-HER2 antibody, anti-VEGF antibody, anti-CD33 antibody, anti-CCR4 antibody, and anti-CD52 antibody.

### ---Cytocide-type anticancer agent---

The cytocide-type anticancer agent means an anticancer agent that kills cancer cells by inhibiting DNA synthesis or cell division.

Examples of the cytocide-type anticancer agent include alkylating agents, antimetabolic argents, topoisomerase inhibitors, mitotic inhibitors, antitumor antibacterial agents, and platinum complex derivatives. Among them, topoisomerase inhibitors and platinum complex derivatives are preferable.

Examples of the alkylating agents include ifosfamide, cyclophosphamide, dacarbazine, temozolomide, nimustine, busulfan, procarbazine, melphalan, and ranimustine.

Examples of the antimetabolic argents include enocitabine, capecitabine, carmofur, cladribine, gemcitabine, cytarabine, cytarabine ocfosfate, tegafur, tegafur·uracil, tegafur·gimeracil·oteracil potassium, doxifluridine, nelarabine, hydroxycarbamide, fluorouracil, fludarabine, pemetrexed, pentostatin, mercaptopurine, and methotrexate.

Examples of the topoisomerase inhibitors include camptothecin and analogues thereof, etoposide, teniposide, doxorubicin, levofloxacin, and ciprofloxacin. Among them, camptothecin and analogues thereof are preferable.

Examples of the analogues of the camptothecin include irinotecan (CPT-11), nogitecan (topotecan), DB67, BNP1350, exatecan, lurtotecan, ST1481, CKD602, 7-ethyl-10-hydroxycamptothecin (SN-38), and pharmacologically acceptable salts thereof.

Among them, irinotecan (CPT-11), nogitecan (topotecan), and 7-ethyl-10-hydroxycamptothecin (SN-38) are preferable.

The camptothecin analogue may be a form of a composite enclosing the camptothecin or the camptothecin analogue therein.

Examples of the composite include a matter obtained by enclosing the camptothecin analogue in a liposome, a composite of the camptothecin analogue and phospholipid, a matter obtained by enclosing the camptothecin analogue in polymer micelles, and a matter obtained by enclosing the camptothecin analogue in a block copolymer formed of a hydrophilic segment and a hydrophobic segment.

Examples of the matter obtained by enclosing the camptothecin analogue in the block copolymer formed of the hydrophilic segment and the hydrophobic segment include a high-molecular derivative of the camptothecin analogue represented by the General Formula (I).

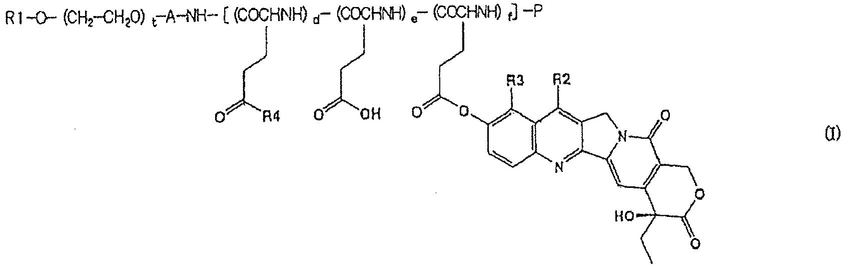

In the General Formula (I), R1 represents a (C1-C6) alkyl group that may have a hydrogen atom or a substituent, t represents an integer of from 5 to 11,500, A represents a linking group, d, e, and f represent an integer, d+e+f represents an integer of from 3 to 200, R2 represents a hydrogen atom, a (C1-C6) alkyl group that may have a substituent, or a silyl group that may have a substituent, R3 represents a hydrogen atom or a (C1-C6) alkyl group that may have a substituent, R4 represents a (C1-C20) alkoxyl group that may have a substituent, a (C1-C20) alkylamino group that may have a substituent, a di(C1-C20) alkylamino group that may have a substituent, or a (C1-C20) alkylaminocarbonyl (C1-C20) alkylamino group that may have a substituent, and P represents a hydrogen atom, a (C1-C6) acyl group, or a (C1-C6) alkoxycarbonyl group.

Examples of the mitotic inhibitors include paclitaxel and analogues thereof, vinblastine, vincristine, and vindesine. Among them, the paclitaxel and the analogues thereof are preferable.

The paclitaxel and the analogues thereof are a compound having a taxane-based structure. Examples thereof include paclitaxel (CAS No. 33069-62-4), 7α-glucosyloxyacetylpaclitaxel, docetaxel, and pharmacologically acceptable salts thereof.

The paclitaxel analogue may be a form of a composite enclosing the paclitaxel or the paclitaxel analogue therein.

Examples of the composite include a matter obtained by combining the paclitaxel analogue with albumin, a matter obtained by enclosing the paclitaxel analogue in a liposome, a composite of the paclitaxel analogue and phospholipid, a matter obtained by enclosing the paclitaxel analogue in polymer micelles, and a matter obtained by enclosing the paclitaxel analogue in a block copolymer formed of a hydrophilic segment and a hydrophobic segment.

Examples of the matter obtained by enclosing the paclitaxel analogue in the block copolymer formed of the hydrophilic segment and the hydrophobic segment include a matter obtained by enclosing the paclitaxel analogue in a block copolymer represented by the General Formula (II).

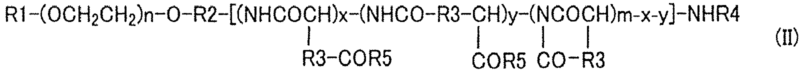

In the General Formula (II), R1 represents a hydrogen atom or a (C1-C5) alkyl group, R2 represents a (C1-C5) alkylene group, R3 represents a methylene group or an ethylene group, R4 represents a hydrogen atom or a (C1-C4) acyl group, R5 represents a hydroxyl group, an aryl (C1-C8) alkoxy group that may have a substituent, or -N(R6)-CO-NHR7, and R6 and R7, which may be identical to or different from each other, represent a (C1-C5) alkyl group that may be substituted with a (C3-C6) cyclic alkyl group or a tertiary amino group. In addition, n is an integer of from 5 to 1,000, m is an integer of from 2 to 300, x is an integer of from 0 to 300, and y is an integer of from 0 to 300. Here, the sum of the x and the y is 1 or more but is equal to or smaller than the m, a rate of R5 being a hydroxyl group is from 0% to 88% of the m, a rate of R5 being an aryl (C1-C8) alkoxy group that may have a substituent is from 1% to 89% of the m, and a rate of R5 being -N(R6)-CO-NHR7 is from 11% to 30% of the m.

Examples of the antitumor antibacterial agents include mitomycin C, bleomycin, adriamycin, and doxorubicin.

Examples of the platinum complex derivatives include cisplatin, carboplatin, and oxaliplatin. Among them, the oxaliplatin is preferable.

When the present invention is performed through an *in vivo* method for evaluating hematotoxicity, the drug to be evaluated may be administered to the transgenic NOG mouse. When the present invention is performed through the *in vitro* method for evaluating hematotoxicity, the drug to be evaluated may be administered to a sample of the transgenic NOG mouse.

The sample is not particularly limited and may be appropriately selected depending on the intended purpose so long as the sample is a sample derived from the transgenic NOG mouse. Examples thereof include bone marrow cells, blood cells, splenocytes, and hepatocytes. Among them, bone marrow cells and blood cells are preferable.

The bone marrow cells can be prepared by the following method, for example.

Specifically, the humanized, transgenic NOG mouse is euthanized by removing the blood from the abdominal aorta under anesthesia of isoflurane. Then, the femur is collected. The femur is sterilely washed using the Iscove's Modified Dulbecco's Medium (IMDM medium) to prepare a suspension of cells. The suspension of cells is passed through a nylon filter (for example, pore size of 35 µm, Cell Strainer, available from BD Biosciences) to obtain a filtrate as a sample including the bone marrow cells.

A method for administering the drug to be evaluated is not particularly limited and conditions of the administration such as a route of the administration, a dose, and a schedule of the administration required for each drug can be appropriately selected depending on the intended purpose.

For example, when nogitecan is evaluated, it is preferable that nogitecan of from 3 mg/kg to 30 mg/kg (for example, 30 mg/kg) be administered through single intravenous administration. When paclitaxel is evaluated, it is preferable that paclitaxel of from 25 mg/kg to 100 mg/kg (for example, 70 mg/kg) be administered through single intravenous administration. When oxaliplatin is evaluated, it is preferable that oxaliplatin of from 2.5 mg/kg to 20 mg/kg (for example, 20 mg/kg) be administered through single intravenous administration.

Alternatively, as a method for administering the drug to be evaluated, conditions of the administration such as a route of the administration, a dose, and a schedule of the administration to be evaluated can be appropriately selected depending on the intended purpose. As a result, it is possible to evaluate each drug for suitable administration conditions (so as to exhibit, for example, low hematotoxicity and excellence in property of recovering) to be selected.

When the present invention is performed through the *in vitro* method for evaluating hematotoxicity, a method for administering the drug to be evaluated is not particularly limited and each condition can be selected from various conditions of *in vitro* known experiments. Specifically, a medium including the drug to be evaluated may be administered to a sample of the transgenic NOG mouse.

### <Step of evaluation>

The step of evaluation includes a step of evaluating that the drug to be evaluated has hematotoxicity in a case where when a sample of the transgenic NOG mouse to which the drug to be evaluated has been administered is measured for the number of human-derived granulocytes and the number of said granulocytes is reduced compared to a control.

### -Sample-

The sample is not particularly limited and may be appropriately selected depending on the intended purpose. Examples thereof include blood, bone marrow cells, splenocytes, and hepatocytes. Among them, blood is preferable.

The blood is not particularly limited and may be appropriately selected depending on the intended purpose. Examples thereof include venous blood and peripheral blood. Among them, peripheral blood is preferable in terms of low stress to be applied to the transgenic NOG mouse (i.e., a subject) and easiness of collection.

### -Cells of the human-derived hemocytes-

The cells of the human-derived hemocytes used by the method of the invention are granulocytes.

According to the invention, human-derived granulocytes are used because differentiation into the human-derived granulocytes, which is not observed in the existing experimental mice, is observed in the transgenic NOG mouse for the first time and thus the evaluation of the human-derived granulocytes can be performed.

When the *in vitro* method for evaluating hematotoxicity is performed, a sample derived from the transgenic NOG mouse, to which the drug to be evaluated is administered, may be measured for the number of human-derived granulocytes.

It is preferable that the number of the cells of the human-derived granulocytes and the number of mouse-derived granulocytes be measured.

The measurement may be performed at one measurement point at which a certain time has passed after the administration, but is preferably performed over time (at a plurality of measurement points) during a predetermined schedule. In other words, the step of measurement is preferably performed by collecting samples of the transgenic NOG mouse after the administration over time and measuring the number of human-derived granulocytes in each of the samples over time.

A schedule of the measurements performed over time is not particularly limited and may be appropriately selected depending on the intended purpose. Examples thereof include schedules such as day 4 after the administration, day 7 after the administration, day 14 after the administration, day 21 after the administration, day 28 after the administration, day 35 after the administration, and day 42 after the administration.

A method for measuring the number of human-derived hemocytes, according to the invention at least granulocytes, in the sample is described below. Specifically, after a certain time has passed after the administration, the blood of the transgenic NOG mouse is collected from the orbital sinus. The total number of the leucocytes in the blood is measured by, for example, an automated hematology analyzer. Then, the number of human-derived hemocytes, according to the invention at least granulocytes, can be measured by calculating the number of human-derived hemocytes, according to the invention at least granulocytes, in the blood based on ratios obtained through fluorescent flow cytometry (FCM) measurement using a surface antigen marker, which is specific to humans, as an indicator.

The number of mouse-derived hemocytes, according to the invention at least granulocytes, can be measured in the same manner as described above except that the aforementioned surface antigen marker is changed to a surface antigen marker, which is specific to the mouse.

The surface antigen marker is not particularly limited and may be appropriately selected from known markers used for detecting cells of hemocytes, according to the invention at least granulocytes, depending on the intended purpose. Examples thereof include:
surface antigen markers relating to the general leucocytes (e.g., CD45);
surface antigen markers relating to the granulocytes (e.g., CD66b, CD16, and Gr-1);
surface antigen markers relating to the B cells: (e.g., CD19); and
surface antigen markers relating to the T cells: (e.g., CD3).

The leucocytes are classified using the following surface antigen markers as an indicator. The calculation of the ratio makes it possible to separately detect the human-derived leucocytes and the mouse-derived leucocytes.
- The cells of the human-derived hemocytes: hCD45⁺ cells (human leucocytes), hCD66b⁺ cells (human granulocytes), hCD66b⁺hCD16⁺ cells (human neutrophils), hCD19⁺ cells (human B cells)
- The cells of the mouse-derived hemocytes: mCD45⁺ cells (mouse leucocytes), mGr-1⁺ cells (mouse granulocytes)

In addition, the total number of the leucocytes in the blood is counted using, for example, an automated hematology analyzer (e.g., XT-2000iV, SYSMEX CORPORATION). Then, the number of each of the cells of the hemocytes, according to the invention at least granulocytes, is calculated based on a ratio obtained from fluorescent flow cytometry (FCM).

The fluorescent flow cytometry (FCM) measurement is not particularly limited and may be appropriately selected from known devices depending on the intended purpose. Examples thereof include FACS (fluorescence activated cell sorting) devices such as LSRII (BD Bioscience).

The erythrocytes are classified using the following surface antigen markers as an indicator. The calculation of the ratio makes it possible to separately detect the human-derived erythrocytes and the mouse-derived erythrocytes.
- The cells of the human-derived hemocytes: hCD235a⁺ cells (human erythrocytes)
- The cells of the mouse-derived hemocytes: mTER119⁺ cells (mouse erythrocytes)

The platelets are classified using the following surface antigen markers as an indicator. The calculation of the ratio makes it possible to separately detect the human-derived platelets and the mouse-derived platelets.
- Human-derived platelets: hCD41a⁺ cells (human platelets)
- Mouse-derived platelets: mCD41⁺ cells (mouse platelets)

### -Control-

The control is a control mouse treated in the same manner as in the transgenic NOG mouse except that the vehicle used for the drug is only administered instead of the drug to be evaluated.

When the present invention is performed through the *in vitro* method for evaluating hematotoxicity, the control is a control sample treated in the same manner as in the manner applied to the sample of the transgenic NOG mouse except that the vehicle used for the drug is only administered instead of the drug.

The vehicle is not particularly limited and may be appropriately selected depending on the drug to be evaluated which is used so long as it can dissolve the drug to be evaluated and is pharmaceutically acceptable. Examples thereof include a physiological saline solution, a 5 w/v% glucose solution, and a physiological saline solution including 5 v/v% ethanol/5 v/v% Cremophor.

### -Evaluation of hematotoxicity-

The evaluation that the drug to be evaluated has hematotoxicity is performed based on at least reduction in the granulocytes, and may further comprise at least one of reduction in the leucocytes, reduction in the erythrocytes, reduction in the platelets, increase in the leucocytes, and reduction in panhemocytes.

The "changed" means statistically significant (for example, student's t-test, p<0.05) increase or decrease relative to the control. The same holds for "reduced".

A degree of the change/reduction is not particularly limited and may be appropriately selected depending on a drug to be evaluated, hematotoxicity, and conditions of the administration. Examples of the degree include 10% or more, 20% or more, 30% or more, 40% or more, 50% or more, 60% or more, 70% or more, 80% or more, and 90% or more.

In addition to presence or absence of hematotoxicity (qualitative evaluation), comparison between degrees of change/reduction in a plurality of measurement points (for example, measurement points that are different in days passed after the administration, a drug, and a dose) makes it possible to perform a qualitative evaluation such as comparison between degrees of strength and weakness of hematotoxicity.

When the number of the cells of the granulocytes is statistically significantly (for example, student's t-test, p<0.05) reduced compared to the control, it is judged that the drug to be evaluated has hematotoxicity.

Differentiation into the human-derived granulocytes, which is not observed in the existing experimental mice, is observed in the transgenic NOG mouse for the first time and thus the evaluation of the human-derived granulocytes can be performed. Therefore, it is preferable that the cells of the human-derived hemocytes be the human-derived granulocytes and the hematotoxicity be the reduction in granulocytes.

### --Reduction in leucocytes--

When the number of the cells of the leucocytes is statistically significantly (for example, student's t-test, p<0.05) reduced compared to the control, it is judged that the drug to be evaluated has hematotoxicity.

### --Reduction in granulocytes-

Reduction/increase of any of the other cell types are optional features of claim 1.

### --Reduction in erythrocytes--

When the number of the cells of the erythrocytes is statistically significantly (for example, student's t-test, p<0.05) reduced compared to the control, it is judged that the drug to be evaluated has hematotoxicity.

### --Reduction in platelets--

When the number of the cells of the platelets is statistically significantly (for example, student's t-test, p<0.05) reduced compared to the control, it is judged that the drug to be evaluated has hematotoxicity.

### --Increase in leucocytes--

When the number of the cells of the leucocytes is statistically significantly (for example, student's t-test, p<0.05) increased compared to the control, it is judged that the drug to be evaluated has hematotoxicity.

The increase in the leucocytes closely relates to abnormal myelopoiesis such as hyperleukocytosis.

### --Reduction in panhemocyte--

When the number of the cells of the leucocytes, the number of the cells of the erythrocytes, and the number of the cells of the platelets are each statistically significantly (for example, student's t-test, p<0.05) reduced compared to the control, it is judged that the drug to be evaluated has hematotoxicity.

In addition to the step of evaluation where the cells of the human-derived hemocytes, according to the invention at least granulocytes, are evaluated, the cells of the mouse-derived hemocytes, in particular granulocytes, are preferably evaluated. Specifically, the method of the present invention further preferably includes a step of evaluating that the drug to be evaluated has hematotoxicity in a case where the number of the cells of the mouse-derived hemocytes, in particular granulocytes, is changed compared to the control.

### <Other steps>

Examples of the other steps include a step of evaluating property of recovering and a step of evaluating a difference between the species.

### «Step of evaluating property of recovering»

The method of the present invention for evaluating hematotoxicity of the drug to be evaluated further preferably includes a step of evaluating property of recovering.

The step of evaluating property of recovering is a step of measuring a period from when the number of human-derived granulocytes was reduced compared to the control until when it recovers to the number before the administering the drug, and evaluating property of recovering based on the period.

This makes it possible to predict a degree of recovery from hematotoxicity and also makes it possible to estimate dose response of the drug and appropriate intervals at which the drug is administered to the human, in human clinical practices. Therefore, physical stress, which is applied to a human receiving an anticancer agent, is reduced. In addition, it is easy to prepare a dosage regimen.

Here, the phrase "returns to the number of the cells before the administration" means that a degree of change relative to the number of human-derived granulocytes before the administration is not statistically significant (for example, student's t-test, p<0.05).

### <<Step of evaluating difference between species>>

Preferably, the method of the present invention for evaluating hematotoxicity of the drug to be evaluated further includes a step of evaluating a difference between the species.

The step of evaluating a difference between the species is a step of evaluating a difference of hematotoxicity between the species based on an evaluation result of the cells of the human-derived granulocytes and an evaluation result of the cells of the mouse-derived granulocytes.

This makes it possible to evaluate a difference of hematotoxicity between the species and also makes it possible to presume evaluation results of humans, which cannot be presumed from the results of the conventional evaluation of hematotoxicity using a mouse different from the transgenic NOG mouse.

The "difference between the species" means that, at the same measurement point, a degree of change of human-derived granulocytes relative to the control and a degree of change of mouse-derived granulocytes relative to the control are statistically significantly (for example, student's t-test, p<0.05) different.

The degree of change is not particularly limited and may be appropriately selected depending on the intended drug, hemocytes, and conditions of the administration. Examples thereof include 10% or more, 20% or more, 30% or more, 40% or more, 50% or more, 60% or more, 70% or more, 80% or more, and 90% or more.

In addition to presence or absence of hematotoxicity (qualitative evaluation), comparison between degrees of change in a plurality of measurement points (for example, measurement points that are different in days passed after the administration, a drug, and a dose) makes it possible to perform a quantitative evaluation.

According to the method of the present invention for evaluating hematotoxicity of the drug to be evaluated, it is possible to provide a method for evaluating hematotoxicity of a drug to be evaluated where the method is excellent in clinical predictability of toxicity to the human bone marrow. In particular, it is possible to provide a method for evaluating hematotoxicity of a drug to be evaluated where the method is excellent in clinical predictability of human granulocytopenia. According of the method of the present invention for evaluating hematotoxicity of the drug to be evaluated, the evaluation of hematotoxicity can be performed by using rodents. Therefore, the evaluation experiment of hematotoxicity using an anthropoid can be drastically reduced. In addition, it is possible to evaluate, for hematotoxicity, drugs such as an anticancer agent that develops its effect in the body through metabolism. It is difficult to perform the aforementioned type of evaluation in *in vitro* evaluation systems. Use of the present invention excellent in human clinical predictability makes it possible to set a dose of the drug for humans, and administration conditions for inhibiting occurrence of hematotoxicity can be selected. According to the present invention, it is possible to consider administration conditions of the drug (e.g., administration route, dose, and administration schedule) and perform analysis of a fluctuation profile over time, which makes it possible to estimate dose response of the drug and appropriate intervals at which the drug is administered to the human in human clinical practices. In addition, it is possible to presume a degree of recovery from hematotoxicity. Therefore, physical stress applied to a human receiving an anticancer agent is reduced. Moreover, it is easy to prepare a dosage regimen.

### Examples

The present invention will be described in more detail by way of the following Examples. However, the present invention should not be construed as being limited to these Examples.

Details of mice etc. used in Examples are as follows.

### (Mouse)

"Tg-NOG mouse": Created by Central Institute for Experimental Animals
- Sex: male and female
- Weeks old at the time of purchase: from 10 weeks old to 18 weeks old
- Weeks old at the time of irradiation of X-rays: from 6 weeks old to 10 weeks old
- Weeks old at the time of transplantation of CD34⁺ cells derived from the human cord blood: from 6 weeks old to 10 weeks old
- Weeks old at the time at which "cytocide-type anticancer agent" started to be administered to the mouse: from 10 weeks old to 18 weeks old

### (Apparatus)

- X-rays irradiation device: MBR-1505R (available from Hitachi Medical Corporation)
- FACS: LSRII (available from BD Biosciences)
- Hematology analyzer: XT-2000iV (available from SYSMEX

### CORPORATION)

### (Reagent)

- Nogitecan: Topotecan (available from Sigma-Aldrich Japan), which was dissolved in a physiological saline solution so as to have a concentration of 3 mg/mL.
- Paclitaxel (PTX): INDENA (Registered Trademark) available from S. p. A, which was dissolved so as to have a concentration of 35 mg/mL in a mixture solution containing equal amounts of dehydrated ethanol and Cremophor-EL. The resultant solution was 10-fold diluted with a physiological saline solution immediately before the administration.
- Oxaliplatin: Oxaliplatin (NK), available from Nippon Kayaku Co., Ltd., was diluted with a 5 w/v% glucose aqueous solution so as to have a concentration of 2 mg/mL, 3 mg/mL, or 4 mg/mL.
- 7-Ethyl-10-hydroxycamptothecin (EHC): available from Tokyo Chemical Industry Co., Ltd.
- Anti-hCD45 antigen, anti-hCD66b antigen, anti-hCD16 antigen, anti-hCD19 antigen, anti-hCD3 antigen, anti-mCD45 antigen, and anti-mGr-1 antigen: available from BioLegend
- Glucose aqueous solution (5 w/v%): Japanese Pharmacopoeia glucose injection (OTSUKA GLUCOSE INJECTION 5%), available from Otsuka Pharmaceutical Factory, Inc.

### (Cells)

- CD34⁺ cells derived from the human cord blood: frozen CD34⁺ cells derived from the human cord blood (single donor), 1×10⁶ cells/vial, purchased from AllCells, LLC

### Example 1 Evaluation of hematotoxicity of nogitecan

Seventeen-week-old Tg-NOG mice (16 males and 2 females) were irradiated with X-rays of 2.5 Gy. Then, the CD34⁺ cells derived from the human cord blood were transplanted at 40,000 cells/body. At 7 week after the transplantation, the peripheral blood was collected from the orbital sinus under anesthesia of isoflurane. Engraftment thereof in the peripheral blood was observed. Results are presented in Table 1.

**Table 1**

| **Identification marker: type of cells of leukocytes** | **Ratio of human leukocytes (number of human leukocytes in mouse peripheral blood)** |
|---|---|
| hCD45⁺: Human leukocytes | From 43.1 to 63.3% (from 500 to 1088/µL) |
| hCD66b⁺: Human granulocytes | From 2.2 to 9.4% (from 13 to 102/µL) |
| hCD66b⁺16⁺: Human neutrophils | From 0.1 to 0.9% (from 1 to 8/µL) |
| hCD19⁺: Human B-lymphocytes | From 80.1 to 91.2% (from 320 to 859/µL) |
| hCD3⁺: Human T-lymphocytes | 0% (0/µL) |
| mCD45⁺: Mouse leukocytes | From 36.3 to 56.4% (from 370 to 1108/µL) |
| mGr-1⁺: Mouse granulocytes | From 66.9 to 89.6% (from 285 to 897/µL) |
| (The mouse granulocytes are nearly equal to the neutrophils) | |

Twelve mice suitable for the evaluation were selected and divided into 2 groups each having 6 mice. The mice in one group received a 5 w/v% glucose solution and the mice in the other group received nogitecan at 30 mg/kg (3 mg/mL, 10 mL/kg) from the tail vein through single intravenous administration. Regarding assignment of the animals, the animals were grouped so that an average value of the number of the human leucocytes and that of the number of the human granulocytes were almost the same.

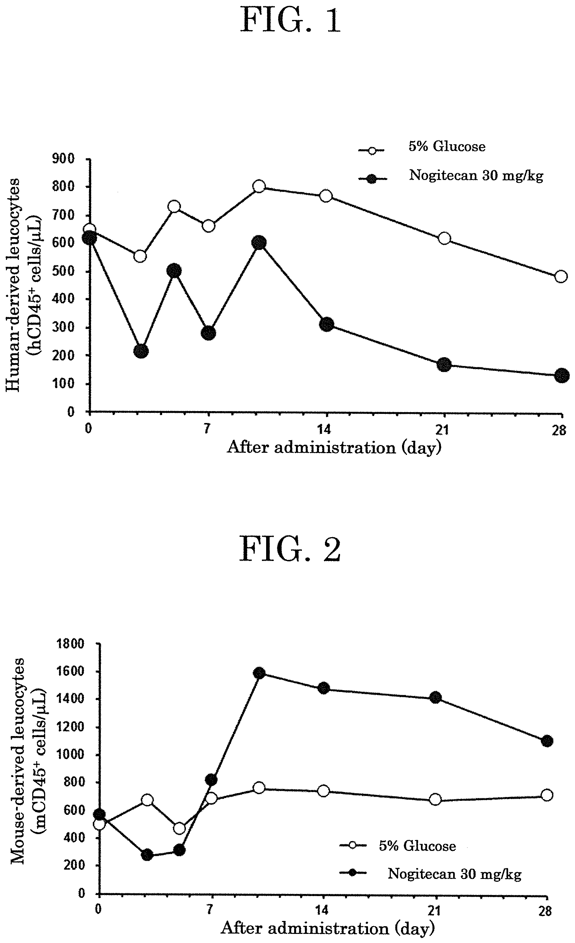

The peripheral blood was collected from the orbital sinus under anesthesia of isoflurane at the day of the administration and at day 3, day 5, day 7, day 10, day 14, day 21, and day 28 after the administration. Then, the number of the leucocytes (WBC) in the peripheral blood was measured with FACS. Results are presented in FIG. 1 to FIG. 4.

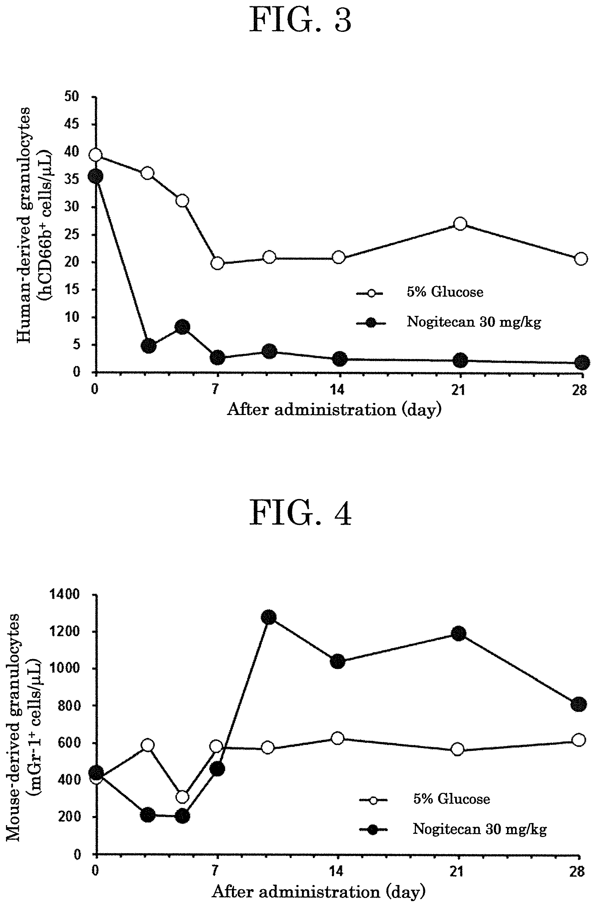

FIG. 1 is a graph presenting a change of the number of the human-derived leucocytes (hCD45⁺) in the evaluation of hematotoxicity of nogitecan. FIG. 2 is a graph presenting a change of the number of the mouse-derived leucocytes (mCD45⁺) in the evaluation of hematotoxicity of nogitecan. FIG. 3 is a graph presenting a change of the number of the human-derived granulocytes (CD66b⁺) in the evaluation of hematotoxicity of nogitecan. FIG. 4 is a graph presenting a change of the number of the mouse-derived granulocytes (Gr-1⁺) in the evaluation of hematotoxicity of nogitecan. In FIG. 1 to FIG. 4, the open circles present the control group (the 5 w/v% glucose administration group) and the closed circles present the group of the administration of nogitecan at 30 mg/kg.

In the Tg-NOG mouse, the number of the mouse-derived leucocytes (mCD45⁺) and the number of the mouse-derived granulocytes (mGr-1⁺) were each the minimum value at a period of from day 3 to day 5 after the administration of nogitecan. However, these numbers started to increase at day 7. The degree of the reduction was about 50% of the value before the administration.

In the Tg-NOG mouse, the number of the human-derived granulocytes (hCD66b⁺) considerably decreased over a period of from day 7 to day 10 after the administration of nogitecan and the number of the granulocytes did not present a tendency of recovering even at day 28 after the administration. As seen from the above, it is found that administration of nogitecan to humans at 30 mg/kg highly possibly causes granulocytopenia.

When the fluctuation of the human leucocytes and that of the mouse leucocytes are separately measured, it is demonstrated that an influence on the reduction in the leucocytes after the administration of nogitecan is more strongly developed in the human than in the mouse. In addition, it is found that this makes it possible to confirm a difference of hematotoxicity between the species.

### Example 2-1 Evaluation of hematotoxicity of PTX

Nine-week-old Tg-NOG mice (35 females) were irradiated with X-rays of 2.5 Gy. After a period of from 3 hours to 5 hours, the CD34⁺ cells derived from the human cord blood were transplanted at 40,000 cells/body. At 5 week after the transplantation, the peripheral blood was collected from the orbital sinus under anesthesia of isoflurane. Engraftment thereof in the peripheral blood was observed. Results are presented in Table 2.

**Table 2**

| **Identification marker: type of cells of leukocytes** | **Ratio of human leukocytes (number of human leukocytes in mouse peripheral blood)** |
|---|---|
| hCD45⁺: Human leukocytes | From 56.0 to 90.7% (300 to 1122/µL |
| hCD66b⁺: Human granulocytes | From 1.4 to 5.6% (4 to 46/µL) |
| hCD66b⁺16⁺: Human neutrophils | From 0.1 to 0.7% (0 to 6/µL) |
| hCD19⁺: Human B-lymphocytes | From 70.6 to 86.5% (255 to 952/µL) |
| hCD3⁺: Human T-lymphocytes | 0% (0/µL) |
| mCD45⁺: Mouse leukocytes | From 9.1 to 44.0% (47 to 493/µL) |
| mGr-1⁺: Mouse granulocytes | From 54.7 to 85.8% (27 to 387/µL) |
| (The mouse granulocytes are nearly equal to the neutrophils) | |

Nine mice suitable for the evaluation were selected and divided into a control group of 5 mice and a PTX administration group of 4 mice. The mice in the control group received a vehicle solution used for PTX (5 v/v% ethanol/5 v/v% Cremophor-including physiological saline solution) and the mice in the PTX administration group received PTX at 70 mg/kg (3.5 mg/mL, 20 mL/kg) from the tail vein through single intravenous administration. Regarding assignment of the animals, the animals were grouped so that an average value of the number of the human leucocytes and that of the number of the human granulocytes were almost the same.

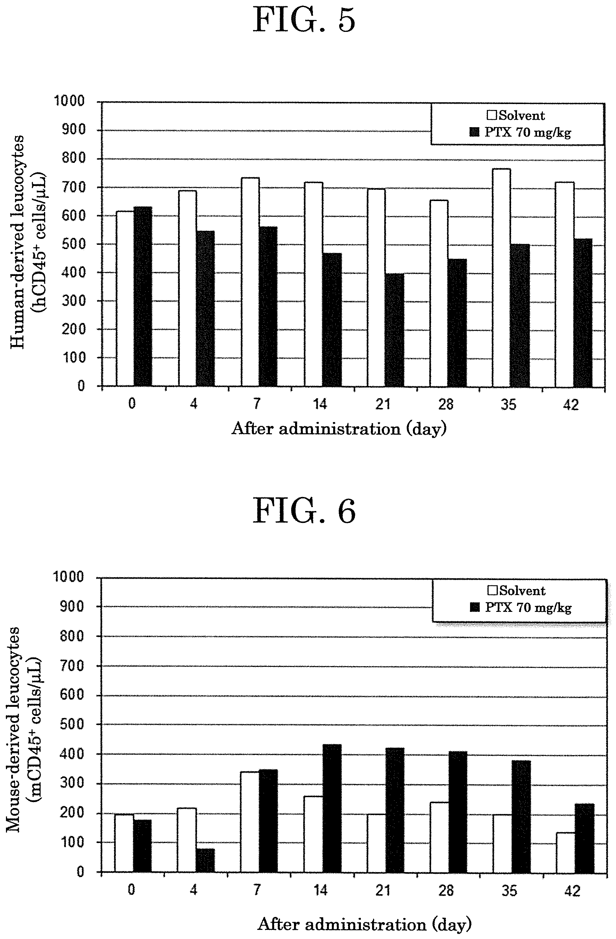

The peripheral blood was collected from the orbital sinus under anesthesia of isoflurane at day 4, day 7, day 14, day 21, day 28, day 35, and day 42 after the administration. Then, the number of the leucocytes in the peripheral blood was measured with FACS. Results are presented in FIG. 5 to FIG. 10.

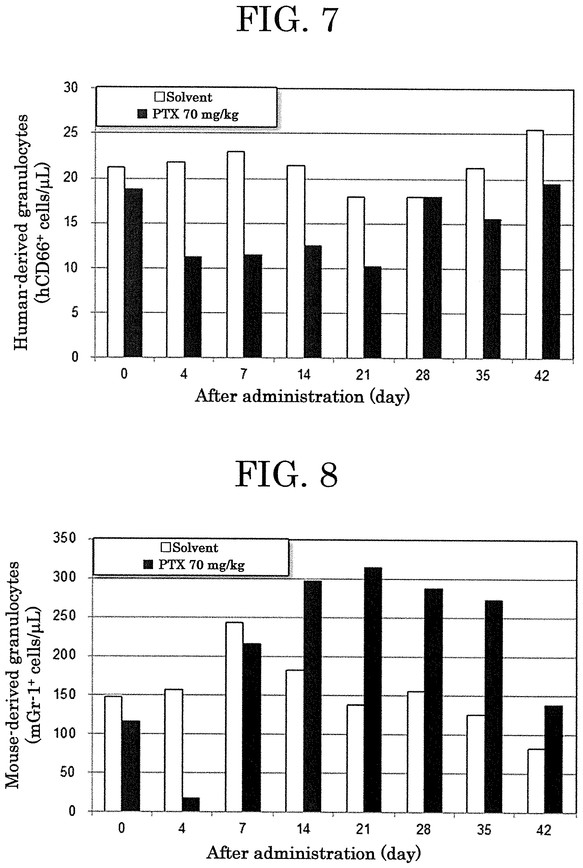

FIG. 5 is a graph presenting a change of the human-derived leucocytes (hCD45+) in the evaluation of hematotoxicity of paclitaxel. FIG. 6 is a graph presenting a change of the number of the mouse-derived leucocytes (mCD45+) in the evaluation of hematotoxicity of paclitaxel. FIG. 7 is a graph presenting a change of the human-derived granulocytes (CD66b+) in the evaluation of hematotoxicity of paclitaxel. FIG. 8 is a graph presenting a change of the number of the mouse-derived granulocytes (Gr-1⁺) in the evaluation of hematotoxicity of paclitaxel. In FIG. 5 to FIG. 8, the open bars present the control group (the group of the administration of 5 v/v% ethanol/5 v/v% Cremophor-including physiological saline solution) and the closed bars present the group of the administration of paclitaxel at 70 mg/kg.

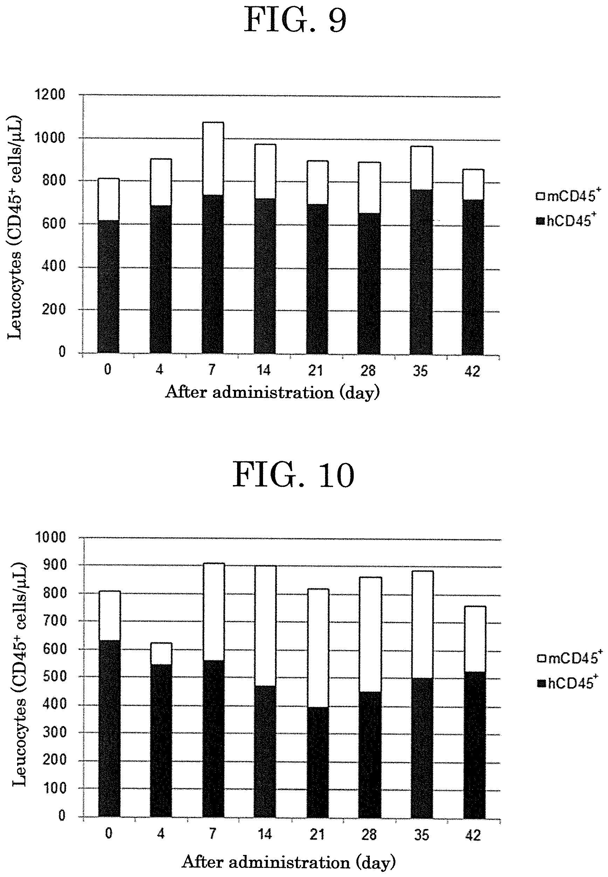

FIG. 9 is a graph presenting a change of a rate between the number of the human-derived leucocytes (hCD45⁺) and the number of the mouse-derived leucocytes (mCD45⁺) of the control group in the evaluation of hematotoxicity of paclitaxel. FIG. 10 is a graph presenting a change of a rate between the number of the human-derived leucocytes (hCD45⁺) and the number of the mouse-derived leucocytes (mCD45⁺) of the paclitaxel-administered group in the evaluation of hematotoxicity of paclitaxel. In FIG. 9 and FIG. 10, the open bars present the number of the mouse-derived leucocytes (mCD45⁺) and the closed bars present the number of the human-derived leucocytes (hCD45⁺).

In the Tg-NOG mouse, the mouse-derived granulocytes (mGr-1⁺) decreased at day 4 after the administration of PTX and started to increase at day 7 after the administration (i.e., property of recovering was observed). The human-derived granulocytes (hCD66b⁺) and the human-derived leucocytes (hCD45⁺) decreased over a period of day 21 after the administration of PTX. The number of the human-derived granulocytes (hCD66b⁺) was the low value at a period of from day 4 to day 21 after the administration and started to increase at day 28 after the administration. Therefore, the property of recovering was demonstrated. As seen from the above, it is found that administration of PTX to the human at 70 mg/kg highly possibly causes granulocytopenia. In addition, when the fluctuation of the human leucocytes and that of the mouse leucocytes after the administration of PTX were separately measured, both of these fluctuations decreased. Therefore, it is found that there is no big difference of reactivity between the species. As understood from the change of the period from the reduction to the recovering, the number of the mouse-derived granulocytes started to increase at day 7 after the administration, while the number of the human-derived granulocytes started to increase at day 28 after the administration. Therefore, it is found that there is a difference of the recovery period between the species. As understood from the above, it is found that use of this model makes it possible to presume a degree of the reduction of the human leucocytes and makes it possible to presume the recovery period as well. Presumption of the recovery period makes it possible to set intervals of the administration for humans.

### Example 2-2 Evaluation of hematotoxicity of oxaliplatin

Six-week-old Tg-NOG mice (24 females) were irradiated with X-rays of 2.5 Gy. After a period of from 3 hours to 4 hours, the CD34⁺ cells derived from the human cord blood were transplanted at 40,000 cells/body. At 5 week after the transplantation, the peripheral blood was collected from the orbital sinus under anesthesia of isoflurane. Engraftment thereof in the peripheral blood was confirmed. Results are presented in Table 3.

**Table 3**

| **Identification marker: type of cells of leukocytes** | **Ratio of human leukocytes (number of human leukocytes in mouse peripheral blood)** |
|---|---|
| hCD45⁺: Human leukocytes | From 9.0 to 44.8% (93 to 381/µL) |
| hCD66b⁺: Human granulocytes | From 4.9 to 13.1% (7 to 29/µL) |
| hCD66b⁺hCD16⁺: Human neutrophils | From 0.3 to 2.0% (0 to 5/µL) |
| hCD19⁺: Human B-lymphocytes | From 36.4 to 68.4% (50 to 195/µL) |
| hCD3⁺: Human T-lymphocytes | 0% (0/µL) |
| mCD45⁺: Mouse leukocytes | From 55.2 to 91.0% (286 to 1302/µL) |
| mGr-1⁺: Mouse granulocytes | From 64.2 to 89.2% (222 to 1080/µL) |
| (The mouse granulocytes are nearly equal to the neutrophils) | |

Eleven mice suitable for the evaluation were selected and divided into the control group (5 mice) and the oxaliplatin administration group (6 mice). The mice in the control group received a 5 w/v% glucose solution from the tail vein through single intravenous administration. Meanwhile, two mice of the 6 mice in the oxaliplatin administration group received oxaliplatin at 20 mg/kg (2 mg/mL, 10 mL/kg), another two mice in the oxaliplatin administration group received oxaliplatin at 30 mg/kg (3 mg/mL, 10 mL/kg), and the other two mice in the oxaliplatin administration group received oxaliplatin at 40 mg/kg (4 mg/mL, 10 mL/kg) from the tail vein through single intravenous administration. Regarding assignment of the animals, the animals were grouped so that an average value of the number of the human leucocytes and that of the number of the human granulocytes were almost the same.

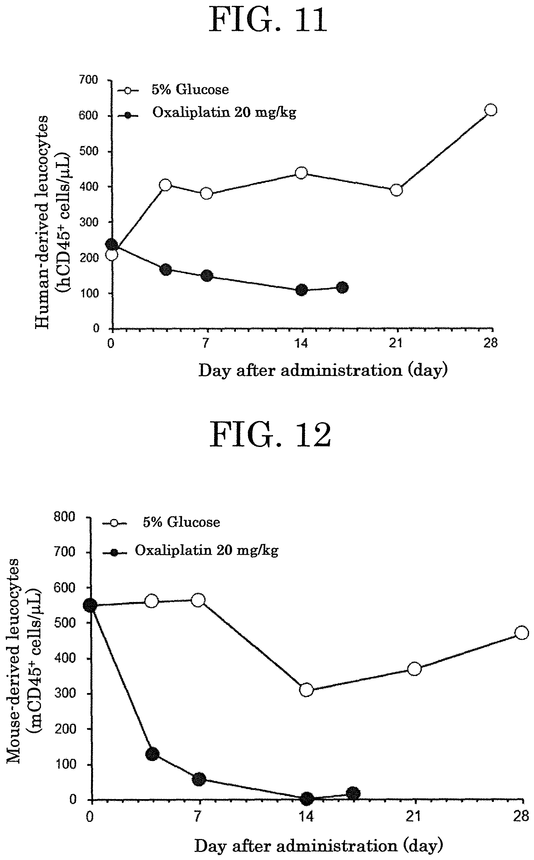

The peripheral blood was collected from the orbital sinus under anesthesia of isoflurane at day 4, day 7, day 14, day 17, day 21, and day 28 after the administration. Then, the number of the leucocytes in the peripheral blood was measured with FACS. The animals administered at 30 mg/kg and the animals administered at 40 mg/kg died by day 7 after the administration. Therefore, results of the animals administered at 20 mg/kg are presented in FIG. 11 to FIG. 14.

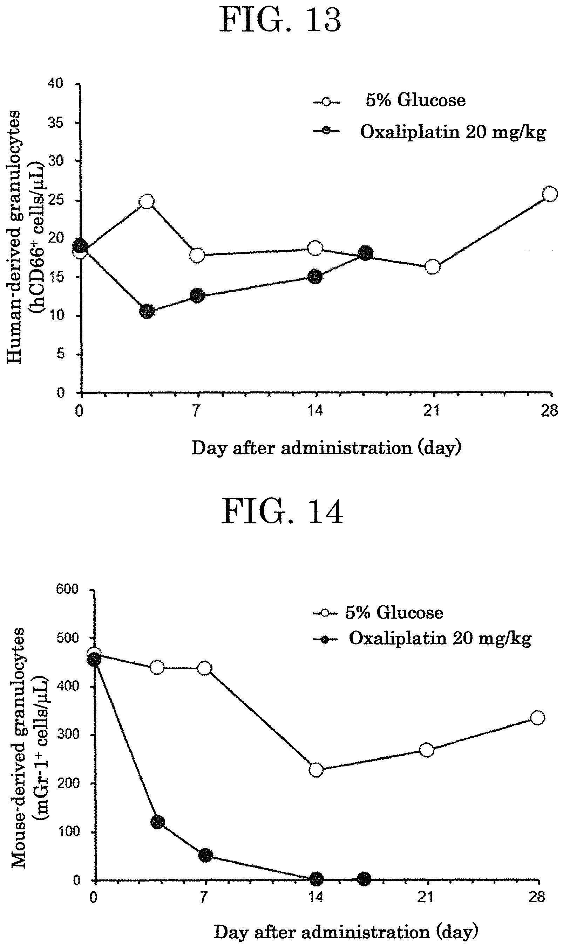

FIG. 11 is a graph presenting a change of human-derived leucocytes (hCD45⁺) in the evaluation of hematotoxicity of oxaliplatin. FIG. 12 is a graph presenting a change of the number of the mouse-derived leucocytes (mCD45⁺) in the evaluation of hematotoxicity of oxaliplatin. FIG. 13 is a graph presenting a change of the number of the human-derived granulocytes (CD66b⁺) in the evaluation of hematotoxicity of oxaliplatin. FIG. 14 is a graph presenting a change of the number of the mouse-derived granulocytes (Gr-1⁺) in the evaluation of hematotoxicity of oxaliplatin. In FIG. 11 to FIG. 14, the open circles present the control group (the 5 w/v% glucose administration group) and the closed circles present the oxaliplatin administration group at 20 mg/kg.

In the NOG mouse, the mouse-derived leucocytes (mCD45⁺) and the mouse-derived granulocytes (mGr-1⁺) considerably decreased after the administration of oxaliplatin and were in a state of the moribund condition at day 17 after the administration without presenting the tendency of recovering.

In the Tg-NOG mouse, the human-derived granulocytes (hCD66⁺) slightly decreased at day 4 after the administration of oxaliplatin and the tendency of recovering was presented day 7 or later. As seen from the above, it is found that even when such a dose that considerably reduces granulocytes in the mouse is administered to human, there is low possibility that granulocytopenia occurs.

When the fluctuation of the human leucocytes and that of the mouse leucocytes are separately measured, it is demonstrated that an influence on the reduction in the leucocytes after the administration of oxaliplatin is more strongly developed in the human than in the mouse. In addition, it is found that this makes it possible to confirm a difference of hematotoxicity between the species which is different from that in the case of nogitecan.

### Example 3-1 Influence of EHC and PTX on granulocytes-based myelopoiesis progenitor cells through colony forming test

The mouse at week 14 after the transplantation of the hCD34⁺ cells derived from the human cord blood was euthanized by removing the blood from the abdominal aorta under anesthesia of isoflurane. Then, the femur was collected. The femur was sterilely washed using the Iscove's Modified Dulbecco's Medium (IMDM, available from Life Technologies) to prepare a suspension of cells. The suspension of cells was passed through a nylon filter (pore size of 35 µm, Cell Strainer, available from BD Biosciences) to obtain a filtrate as a solution of the bone marrow cells. A portion of the solution of the bone marrow cells was subjected to FACS analysis to calculate a ratio between the human-derived leucocytes (hCD45⁺) and the mouse-derived leucocytes (mCD45⁺).

The human 45⁺ cells were suspended in a methyl cellulose medium (MethoCult H4534, available from Stem cell technologies) so as to be 5×10⁴ cells/1.1 mL/dish. Then, EHC was added at 0.14 nM, 0.4 nM, 1.2 nM, 3.7 nM, 11 nM, or 33 nM. Meanwhile, PTX was added at 0.12 nM, 0.4 nM, 1.1 nM, 3.3 nM, 10 nM, 30 nM, or 90 nM. The resultant was seeded in a dish and was cultured for 16 days at 37°C under 5%CO₂.

The mouse CD45⁺ cells were suspended in a methyl cellulose medium (available from MethoCult GF M3534, Stem cell technologies) so as to be 2×10⁴ cells/1.1 mL/dish. Then, EHC (1.2 nM, 3.7 nM, 11 nM, 33 nM, 100 nM, or 300 nM) was added. Meanwhile, PTX (0.12 nM, 0.4 nM, 1.1 nM, 3.3 nM, 10 nM, 30 nM, or 90 nM) was added. The resultant was seeded in a dish and was cultured for 8 days at 37°C under 5%CO₂.

As the vehicle control, dimethyl sulfoxide (DMSO, final concentration of 0.002 v/v%) was used. For each concentration, two dishes were used.

After a period of the cultivation was completed, the number of the colonies derived from the granulocytes formed was counted and an average number of the colonies in each concentration was calculated. An average number of the colonies in the vehicle control was considered as 100%. The average number of the colonies in each concentration was considered as a relative value (IC50 and IC90). Here, the IC50 means a concentration at which formation of 50% of the colonies is inhibited and the IC90 means a concentration at which formation of 90% of the colonies is inhibited. Results are presented in Table 4.

**Table 4**

| Drug | IC50 (nM) | | Ratio mouse/human | IC90 (nM) | | Ratio mouse/human |
|---|---|---|---|---|---|---|
| | hCD45⁺ | mCD45⁺ | | hCD45⁺ | mCD45⁺ | |
| EHC | 11.3 | 105.9 | 9.3 | 12.9 | 122.2 | 9.5 |
| PTX | 83.9 | 71.2 | 0.8 | 84.5 | 87.5 | 1.0 |

It was found that an influence of EHC on the granulocytes-based progenitor cells was higher in the human than in the mouse. This result is in agreement with a big difference in reactivity between the mouse leucocytes and the human leucocytes as described below. Specifically, in the analysis results of the peripheral blood, the mouse leucocytes were temporarily reduced and then started to increased, exhibiting the property of recovering. Meanwhile, no property of recovering from the reduction was found in the human leucocytes. An influence of PTX on the progenitor cells of the granulocytes in the bone marrow was almost the same between the human and the mouse. This is in agreement with the fact that the reduction of the human leucocytes was almost the same as that of the mouse leucocytes, in the analysis results obtained from peripheral blood. Example 3-2 Influence of nogitecan and oxaliplatin on granulocytes-based myelopoiesis progenitor cells through colony forming test

The collection, preparation, and cultivation of the bone marrow cells and the calculation of the percentage of inhibition of colony formation were performed in the same manner as in Example 3-1.

The human CD45⁺ cells were exposed to nogitecan at an exposure concentration of 0.41 nM, 1.2 nM, 3.7 nM, 11.1 nM, 33.3 nM, or 100 nM. Meanwhile, the other human CD45⁺ cells were exposed to oxaliplatin at an exposure concentration of 123 nM, 370 nM, 1,111 nM, 3,333 nM, 10,000 nM, or 30,000 nM.

The mouse CD45⁺ cells were exposed to nogitecan at an exposure concentration of 3.7 nM, 11.1 nM, 33.3 nM, 100 nM, 300 nM, or 900 nM. Meanwhile, the other mouse CD45⁺ cells were exposed to oxaliplatin at an exposure concentration of 13.7 nM, 41.2 nM, 123 nM, 370 nM, or 1,111 nM.

A physiological saline solution was used for the vehicle control of nogitecan and a distilled water was used for the vehicle control of oxaliplatin.

An average number of the colonies in the vehicle control was considered as 100%. The average number of the colonies in each concentration was considered as a relative value (IC50 and IC90). Here, the IC50 means a concentration at which formation of 50% of the colonies is inhibited and the IC90 means a concentration at which formation of 90% of the colonies is inhibited. Results are presented in Table 5.

**Table 5**

| Drug | IC50 (nM) | | Ratio mouse/human | IC90 (nM) | | Ratio mouse/human |
|---|---|---|---|---|---|---|
| | hCD45⁺ | mCD45⁺ | | hCD45⁺ | mCD45⁺ | |
| Nogitecan | 8.7 | 141.6 | 16.3 | 24.3 | 304.6 | 12.5 |
| Oxaliplatin | 2765.3 | 434.2 | 0.16 | 4663.4 | 846.0 | 0.18 |

It was presented that an influence of nogitecan on the progenitor cells of the granulocytes in the bone marrow was higher in the human than in the mouse. This tendency was the same as the tendency observed in EHC. This result is in agreement with the facts that after the administration of nogitecan, the human leucocytes in the peripheral blood of the Tg-NOG mouse did not recover from the considerable reduction and a big difference between a change of fluctuation of the mouse leucocytes and a change of fluctuation of the human leucocytes was observed.

It was presented that an influence of oxaliplatin on the progenitor cells of the granulocytes in the bone marrow was higher in the mouse than in the human, which is an opposite tendency observed in those of nogitecan and EHC. This result is in agreement with the fact that after the administration of oxaliplatin, no considerable reduction in the human leucocytes in the peripheral blood of the Tg-NOG mouse was observed, while considerable reduction in the mouse leucocytes was found, which demonstrated that a big difference of reactivity between the species was observed.

As understood from the above, a change of fluctuation of the human leucocytes fraction and a change of fluctuation of the mouse leucocytes fraction in the peripheral blood of the Tg-NOG mouse reflect an influence on the human/mouse hemopoietic progenitor cells in the bone marrow. Therefore, it is demonstrated that the Tg-NOG mouse is useful for a model for evaluating hematotoxicity, which is caused due to the toxicity to the bone marrow at the time of the administration of the anticancer agent, and is also useful for preparation of a dosage regimen.

It was demonstrated that the Tg-NOG mouse is a model capable of evaluating reactivity of hematotoxicity, property of recovering from the hematotoxicity, and a difference of sensitivity between the species by analyzing responsiveness of the drug to the human-derived leucocytes and responsiveness of the drug to the mouse-derived leucocytes, where differentiation into the human-derived leucocytes and the mouse-derived leucocytes is maintained in the body of the mouse.

Therefore, it is found that use of the humanized, transgenic NOG mouse makes it possible to appropriately evaluate reactivity and property of recovering of hematotoxicity on the cells of the human-derived hemocytes, which are different from those on the cells of the mouse-derived hemocytes, without limiting to the specific drug to be evaluated. According to the present invention, it is possible to provide a method for evaluating hematotoxicity of a drug to be evaluated where the method is excellent in clinical predictability of toxicity to the bone marrow for humans.

## Claims

1. A method for evaluating hematotoxicity of a drug to be evaluated, the method comprising:
administering the drug to be evaluated to a humanized, transgenic NOG mouse where the humanized, transgenic NOG mouse is obtained by introducing a human GM-CSF/IL-3 gene combination into a NOG mouse, and the number of human-derived leucocytes in leucocytes in blood thereof is 20% or more; and
evaluating that the drug to be evaluated has the hematotoxicity in a case where when a sample of the humanized, transgenic NOG mouse after administering the drug to be evaluated is measured for the number of human-derived granulocytes, the number of said granulocytes is reduced compared to a control to which a vehicle is administered instead of the drug to be evaluated.

2. The method for evaluating the hematotoxicity of the drug to be evaluated according to claim 1, wherein the drug to be evaluated is an anticancer agent.

3. The method for evaluating the hematotoxicity of the drug to be evaluated according to claim 2, wherein the anticancer agent is a cytocide-type anticancer agent.

4. The method for evaluating the hematotoxicity of the drug to be evaluated according to claim 3, wherein the cytocide-type anticancer agent is at least one of camptothecin analogues, composites including a camptothecin analogue therein, paclitaxel analogues, composites including paclitaxel therein, and platinum complex derivatives.

5. The method for evaluating the hematotoxicity of the drug to be evaluated according to claim 3, wherein the cytocide-type anticancer agent is at least one of irinotecan, nogitecan, 7-ethyl-10-hydroxycamptothecin, paclitaxel, and oxaliplatin.

6. The method for evaluating the hematotoxicity of the drug to be evaluated according to any one of claims 1 to 5, the method further comprising:
measuring a period from when the number of the human-derived granulocytes is reduced compared to the control until when it recovers to the number before the administering the drug, and evaluating property of recovering based on the period.

7. The method for evaluating the hematotoxicity of the drug to be evaluated according to any one of claims 1 to 6, the method further comprising:
measuring the numbers of mouse-derived granulocytes, and evaluating that the drug to be evaluated has the hematotoxicity in a case where when the number of the cells of the mouse-derived granulocytes is reduced compared to the control to which the vehicle is administered instead of the drug to be evaluated; and
evaluating a difference of hematotoxicity of the drug to be evaluated between species based on an evaluation result of the cells of the human-derived granulocytes and an evaluation result of the cells of the mouse-derived granulocytes.

## Patentansprüche

1. Verfahren zur Bewertung der Hämatotoxizität eines zu bewertenden Arzneimittels, wobei das Verfahren umfasst:
Verabreichung des zu bewertenden Arzneimittels an eine humanisierte, transgene NOG-Maus, wobei die humanisierte, transgene NOG-Maus durch Einführen einer menschlichen GM-CSF/IL-3-Genkombination in eine NOG-Maus erhalten wird und die Anzahl der vom Menschen stammenden Leukozyten in den Leukozyten in deren Blut 20% oder mehr beträgt; und
Bewertung, dass das zu bewertende Arzneimittel die Hämatotoxizität aufweist, in einem Fall, bei dem, wenn eine Probe der humanisierten, transgenen NOG-Maus nach Verabreichung des zu bewertenden Arzneimittels auf die Anzahl der vom Menschen stammenden Granulozyten untersucht wird, die Anzahl dieser Granulozyten im Vergleich zu einer Kontrolle, der anstelle des zu bewertenden Arzneimittels ein Vehikel verabreicht wird, reduziert ist.

2. Verfahren zur Bewertung der Hämatotoxizität des zu bewertenden Arzneimittels nach Anspruch 1, wobei das zu bewertende Arzneimittel ein Antikrebsmittel ist.

3. Verfahren zur Bewertung der Hämatotoxizität des zu bewertenden Arzneimittels nach Anspruch 2, wobei das Antikrebsmittel ein Antikrebsmittel vom Cytocid-Typ ist.

4. Verfahren zur Bewertung der Hämatotoxizität des zu bewertenden Arzneimittels nach Anspruch 3, wobei das Antikrebsmittel vom Cytocid-Typ mindestens eines der folgenden ist: Camptothecin-Analoga, Kombinationsstoffe, die ein Camptothecin-Analogon enthalten, Paclitaxel-Analoga, Kombinationsstoffe, die Paclitaxel enthalten, und Platinkomplex-Derivate.

5. Verfahren zur Bewertung der Hämatotoxizität des zu bewertenden Arzneimittels nach Anspruch 3, wobei das Antikrebsmittel vom Cytocid-Typ mindestens eines von Irinotecan, Nogitecan, 7-Ethyl-10-hydroxycamptothecin, Paclitaxel und Oxaliplatin ist.

6. Verfahren zur Bewertung der Hämatotoxizität des zu bewertenden Arzneimittels nach einem der Ansprüche 1 bis 5, wobei das Verfahren ferner umfasst:
Messung eines Zeitraums, in dem die Anzahl der vom Menschen stammenden Granulozyten im Vergleich zur Kontrolle reduziert ist, bis zu dem Zeitpunkt, zu dem sie sich auf die Anzahl vor der Verabreichung des Arzneimittels regeneriert hat, und Bewertung der Eigenschaft, sich zu regenerieren, auf der Grundlage des Zeitraums.

7. Verfahren zur Bewertung der Hämatotoxizität des zu bewertenden Arzneimittels nach einem der Ansprüche 1 bis 6, wobei das Verfahren ferner umfaßt:
Messen der Anzahl der von der Maus stammenden Granulozyten und Bewerten, dass das zu bewertende Arzneimittel die Hämatotoxizität aufweist, in einem Fall, bei dem die Anzahl der Zellen der von der Maus stammenden Granulozyten im Vergleich zu der Kontrolle, der das Vehikel anstelle des zu bewertenden Arzneimittels verabreicht wird, reduziert ist; und
Bewertung eines Unterschieds der Hämatotoxizität des zu bewertenden Arzneimittels zwischen den Arten auf der Grundlage eines Bewertungsergebnisses der Zellen der vom Menschen stammenden Granulozyten und eines Bewertungsergebnisses der Zellen der von der Maus stammenden Granulozyten.

## Revendications

1. Procédé pour évaluer l'hématotoxicité d'un médicament à évaluer, le procédé comprenant :
- l'administration du médicament à évaluer à une souris NOG humanisée, transgénique, la souris NOG humanisée, transgénique étant obtenue par introduction d'une combinaison génique humaine de GM-CSF/IL-3 dans une souris NOG et le nombre de leucocytes d'origine humaine dans le sang de celle-ci étant de 20% ou plus ; et
- l'évaluation du fait que le médicament à évaluer présente une hématotoxicité dans le cas où, lorsque le nombre de granulocytes d'origine humaine est mesuré dans un échantillon de la souris NOG humanisée, transgénique après administration du médicament à évaluer, le nombre desdits granulocytes est réduit par rapport à un témoin auquel un véhicule est administré au lieu du médicament à évaluer.

2. Procédé pour évaluer l'hématotoxicité du médicament à évaluer selon la revendication 1, le médicament à évaluer étant un agent anticancéreux.

3. Procédé pour évaluer l'hématotoxicité du médicament à évaluer selon la revendication 2, l'agent anticancéreux étant un agent anticancéreux de type cytocide.

4. Procédé pour évaluer l'hématotoxicité du médicament à évaluer selon la revendication 3, l'agent anticancéreux de type cytocide étant au moins l'un parmi les analogues de camptothécine, les composites comprenant un analogue de camptothécine en leur sein, les analogues de paclitaxel, les composites comprenant du paclitaxel en leur sein et les dérivés complexes du platine.

5. Procédé pour évaluer l'hématotoxicité du médicament à évaluer selon la revendication 3, l'agent anticancéreux de type cytocide étant au moins l'un parmi l'irinotécan, le nogitécan, la 7-éthyl-10-hydroxycamptothécine, le paclitaxel et l'oxaliplatine.

6. Procédé pour évaluer l'hématotoxicité du médicament à évaluer selon l'une quelconque des revendications 1 à 5, le procédé comprenant en outre :
- la mesure d'une période à partir du moment où le nombre de granulocytes d'origine humaine est réduit par rapport au témoin jusqu'au moment où il rattrape le nombre d'avant l'administration du médicament et l'évaluation de la propriété de rattrapage sur la base de la période.

7. Procédé pour évaluer l'hématotoxicité du médicament à évaluer selon l'une quelconque des revendications 1 à 6, le procédé comprenant en outre :
- la mesure des nombres de granulocytes provenant de souris et l'évaluation du fait que le médicament à évaluer présente une hématotoxicité dans un cas où le nombre de cellules des granulocytes provenant de souris est réduit par rapport au témoin auquel le véhicule est administré au lieu du médicament à évaluer ; et
- l'évaluation d'une différence d'hématotoxicité du médicament à évaluer entre les espèces sur la base d'un résultat d'évaluation des cellules des granulocytes d'origine humaine et d'un résultat d'évaluation des cellules de granulocytes provenant de souris.
